# EUROPÄISCHE PATENTSCHRIFT

(11) **EP 1 135 376 B1**
(45) Veröffentlichungstag und Bekanntmachung des Hinweises auf die Patenterteilung: **17.09.2003**
(21) Anmeldenummer: 99959326.2
(22) Anmeldetag: 17.11.1999
(51) Int. Cl.: C07D 257/02

(54) **VERFAHREN ZUR HERSTELLUNG VON CYCLEN**
METHOD OF PRODUCING CYCLENE
PROCEDE DE PREPARATION DE CYCLENE

(30) Priorität: 02.12.1998 DE 19856481
(43) Veröffentlichungstag der Anmeldung: 26.09.2001
(73) Patentinhaber: SCHERING AKTIENGESELLSCHAFT, 13342 Berlin (DE)
(72) Erfinder: PLATZEK, Johannes, D-12621 Berlin (DE); HOYER, Karsten, D-13505 Berlin (DE); GRASKE, Klaus-Dieter, D-12169 Berlin (DE); RADÜCHEL, Bernd, D-13465 Berlin (DE)
(86) Internationale Anmeldenummer: EP9909089
(87) Internationale Veröffentlichungsnummer: WO00032581

(56) Entgegenhaltungen:
- WO-A-96/28432
- WO-A-97/49691
- WO-A-98/49151
- HERVE G. ET AL.: "Condensation of glyoxal with triethylenetetramine. Stereochemistry, cyclization and deprotection." TETRAHEDRON LETTERS., Bd. 40, Nr. 13, 26. März 1999 (1999-03-26), Seiten 2517-2520, XP002132043 OXFORD GB
- CHEMICAL ABSTRACTS, vol. 131, no. 17, 25. Oktober 1999 (1999-10-25) Columbus, Ohio, US; abstract no. 228667, MO Z. ET AL.: "Recent developments in the synthesis of 1,4,7,10-tetraazacyclododecane" XP002132044 & HUAXUE SHIJI, Bd. 21, Nr. 4, 1999, Seiten 218-219,

## Beschreibung

Die Erfindung betrifft den in den Patentansprüchen gekennzeichneten Gegenstand, d.h. ein Verfahren zur Herstellung von Cyclen.

Cyclen (1,4,7,10-Tetraazacyclododecan) ist ein häufig verwendetes Ausgangsmaterial bei der Herstellung makrocyclischer Komplexbildner und wird vor allem im Bereich der Kernresonanztomographie als Ligand für Gadolinium eingesetzt. Mit ProHance® von Bristol-Myers-Squibb und Dotarem® von Guerbet sind bereits zwei Präparate kommerziell erhältlich. Auch eigene Forschungs- und Entwicklungsprojekte verwenden Cyclen als Ausgangsmaterial. Es besteht daher ein Bedarf an einem bequemen und kostengünstigen Verfahren zur Herstellung dieses Eduktes.

Eines der ersten veröffentlichten Verfahren (Richman und Atkins, J. Am. Chem. Soc. **1974**, 96, P. 2268) bedient sich der Cyclisierung eines Natriumbissulfonamides mit einem entsprechend funktionalisierten Diethylensulfonamid. Weisman und Reed (J. Org. Chem. **1996,** 61, P. 5186 - 5187) benutzen in ihrer Synthese die Reaktion eines Bisthioimidoesters mit Triethylentetramin zum Aufbau eines tricyclischen Bisimins, welches schließlich nach Reduktion zu Cyclen hydrolysiert wird.

Einen indirekteren Weg zu Cyclen beschreiten die Verfahren von V.Panetta et. al. (Tetrahedron Lett. **1992,** Vol. 33, No. 38, P. 5505 - 5508), die eine Cyclisierung eines Tetra-trifluormethansulfonsäureamids des Triethylentetramin mit 1,2-Dibromethan durchführen. Der letzte Reaktionsschritt umfaßt die Freisetzung des Cyclens. Auf eine derartige Vorgehensweise ist auch das Verfahren der Firma Nycomed (WO 96/28433) nach Herstellung von Tribenzylcyclen angewiesen. Die Synthese verläuft über die Reaktion eines geeigneten Triamins mit einem Monoamin oder die zweier geeigneter Diamine.

Zu Tetrabenzylcyclen führt auch das in DE 19608307 offenbarte Verfahren, das eine Tetramerisierung von N-Benzylaziridin als Schlüsselschritt beinhaltet.

Die Firma Dow Chemical nutzt, wie in WO 97/31005 und US 5,587,451 beschrieben, ein Bisimidazolin ausgehend von Triethylentetramin als Intermediat. Der Ringschluß zum tetracyclischen Zwischenprodukt erfolgt mit 1,2-Dibromethan. Die anschließende Hydrolyse legt das Cyclen frei.

Die Firma Bracco nutzt, wie in WO 97/49691 beschrieben einen direkten Weg zu Cyclen, der mit der Kondensation von Triethylentetramin mit Glyoxal - die bereits von Weisman et. al. (Tetrahedron Lett. **1980**, Vol.21, P. 335 - 338) veröffentlicht wurde - startet. Anschließend wird diese durch Reaktion mit 1,2-Dibromethan in eine tetracyclische Zwischenverbindung überführt. Die Entfernung der die vier Heteroatome verbindenden Ethylenbrücke wird durch Oxidation mit Brom mit sich anschließender Hydrolyse (oder auch durch Hydrolyse mit einem primären Diamin, WO 98/49151 )vorgenommen. Die Gesamtausbeute wird mit 25% angegeben.

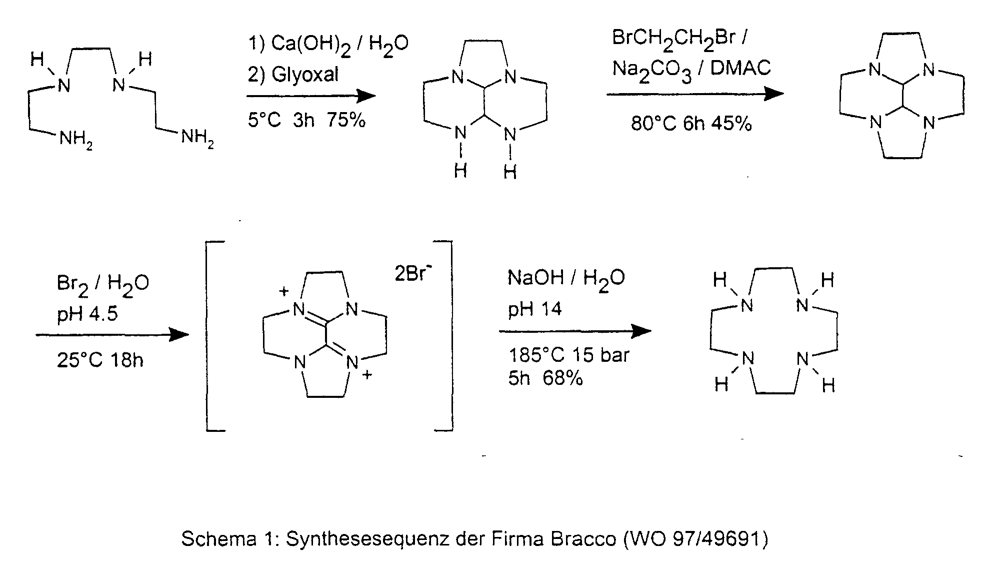

Die in WO 96/28432 offenbarte Synthese der Firma Nycomed ähnelt der oben beschriebenen Sequenz mit dem entscheidenden Unterschied in der Hydrolyse der zentralen Ethylenbrücke. Hier wird die Umsetzung durch Zugabe von Hydroxylamin in ethanolischer Lösung unter Wärme erreicht. Die Gesamtausbeute für diese Reaktionssequenz liegt bei 45%.

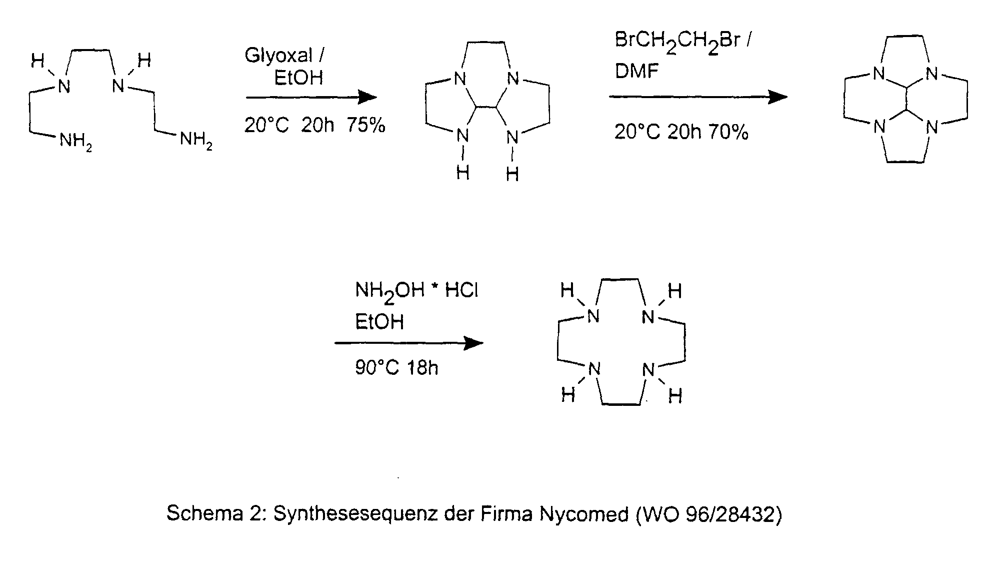

### Bewertuno der Verfahren:

Das Verfahren nach WO 97/49691 weist, durch experimentelle Nachbearbeitung gestützt, einige entscheidende Nachteile auf, die nachfolgend kurz zusammengefaßt sind:
Die Herstellung des Tricyclus ist wie beschrieben nicht reproduzierbar, denn:
   - Das Calciumhydroxid kann nicht quantitativ abgetrennt werden.
   - Größere Mengen Wasser müssen abdestilliert werden.
   - Das Produkt fällt nicht wie angegeben als Öl an.
Die Aufreinigung des Tetracyclus ist sehr aufwendig:
   - Die Extraktion des Produktes aus einem Feststoff schmälert die Ausbeute.
Die Hydrolyse zum Cyclen erweist sich als sehr schwierig:
   - Es muß eine Autoklavenreaktion bei pH = 14 und bei 185°C durchgeführt werden.
   - Das Produkt kristallisiert schlecht und nur sehr verunreinigt aus der Reaktionslösung.

Auch das Verfahren nach WO 96/28432 gibt Anlaß zur Kritik. Die wesentlichen Nachteile sind nachfolgend zusammengestellt:
- Alle Synthesestufen weisen lange Rührzeiten auf.
- Die Aufreinigung des Tetracyclus erfolgt über eine präparative Säulenchromatographie
- Die Hydrolyse zum Cyclen dauert sehr lange und die angegebene Aufreinigungsmethode liefert das Produkt nicht in der gewünschten Reinheit.

Alle übrigen Verfahren umfassen mehrstufige Synthesesequenzen, bei denen Zwischenprodukte isoliert werden, was in der Regel zeit- und rohstoffaufwendig ist. Das Verfahren von Weismann und Reed scheidet für eine kommerzielle Synthese aus, da es auf Rubeanwasserstoff (ca. DM 400,- / 100 g) als eines der Ausgangsmaterialien angewiesen ist. Bei den Verfahren von Richman und Atkins sowie V.Panetta et. al. müssen zuerst entsprechend geschützte Amine präpariert werden. Nach beendeter Reaktion ist dann, wie auch bei den Verfahren der Firmen Dow Chemical, Nycomed (WO 96/28433) und Schering (DE19608307), das Abspalten dieser Schutzgruppen als zusätzlicher Reaktionsschritt erforderlich, der eine schlechtere Stoffbilanz hinsichtlich des gewünschten Produktes bewirkt. Bei der Tetramerisierung von Benzylaziridin muß mit großen Mengen kanzerogener Stoffe gearbeitet werden.

Ein rentables Verfahren sollte auf möglichst preiswerte, umweltschonende und gut zugängliche Rohstoffe zurückgreifen. Ferner sollten die Reaktionszeiten kurz und unter wenig Energieeinsatz ablaufen. Außerden sollten die Stoffmengen während der Gesamtsynthese möglichst gering sein.

Diese Aufgabe wird durch die vorliegende Erfindung gelöst. Es wurde gefunden, daß ein Verfahren zur Herstellung von Cyclen 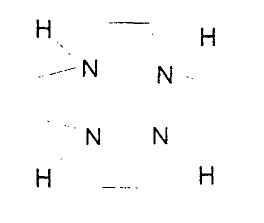 dadurch gekennzeichnet, daß in einem Eintopfverfahren Triethylentetramin mit 40 %igem Glyoxal bei 20 °C bis 80 °C, bevorzugt bei 20° bis 40°C, in einem polaren protischen Lösungsmittel, bevorzugt Methanol, Ethanol, Isopropanol, Butanol, Glykol, Wasser oder deren Mischungen, besonders bevorzugt Ethanol, innerhalb von 4 bis 40 Stunden, bevorzugt 15 bis 20 Stunden, umgesetzt wird, der so gebildete intermediäre Tricyclus nach Entfernen des Lösungsmittels mit einem 1,2-difunktionalisierten Alkylierungsmittel X(CH₂)₂X, worin X für eine nucleofuge Gruppe steht, bevorzugt mit 1,2-Dibromethan, 1,2-Dichlorethan, 1,2-Ditosylethan, 1,2-Dimesylethan oder 1,2-Diiodethan, besonders bevorzugt mit 1,2-Dichlorethan in einem polaren aprotischen Lösungsmittel, bevorzugt in N,N-Dimethylformamid (DMF), N,N-Dimethylacetamid (DMAC), N-Methylpyrrolidon (NMP), Tetramethylharnstoff, Formamid oder Dimethylpropylenharnstoff (DMPU), besonders bevorzugt in DMF, gegebenenfalls in Gegenwart einer Hilfsbase, bevorzugt Natriumcarbonat, Kaliumcarbonat, Calciumcarbonat, Natriumhydrogencarbonat, Kaliumhydrogencarbonat, Magnesiumcarbonat, Magnesiumhydrogencarbonat, Lithiumhydroxid oder Lithiumcarbonat, besonders bevorzugt ohne Hilfsbase, bei 20 bis 120 °, bevorzugt 30 bis 70° C, innerhalb von 2 bis 24 Stunden, bevorzugt 6 bis 10 Stunden, an den beiden sekundären Amin-Stickstoffen alkyliert wird, das so erhaltene Kondensationsprodukt nach Entfernen des Lösungsmittels mit Hydrazinhydrat in einem polaren protischen Lösungsmittel, bevorzugt Methanol, Ethanol, Isopropanol, Butanol, Glykol, Wasser oder deren Mischungen, besonders bevorzugt Ethanol, bei einem pH von 3 bis 6, bevorzugt 3 bis 4, 12 bis 48 Stunden, bevorzugt 25 bis 35 Stunden, bei Rückflußtemperatur behandelt wird, anschließend das Cyclen durch Zugabe einer Base, bevorzugt Natriumhydroxid, Kaliumhydroxid, Calciumhydroxid oder ein basischer Ionenaustauscher, besonders bevorzugt Natriumhydroxid und Kaliumhydroxid, aus dem Cyclensalz freigesetzt wird und nach Eindampfen der Reaktionslösung zur Trockne isoliert wird,
überraschenderweise die oben genannte Aufgabe löst.

Bevorzugt erfolgt die Isolierung des Cyclens durch Kristallisation aus Toluol, Trifluormethylbenzol oder Diethoxymethan, wobei letzteres besonders bevorzugt ist.

Schema 3 verdeutlicht beispielsmäßig nochmals den Verlauf der erfindungsgemäßen Synthese:

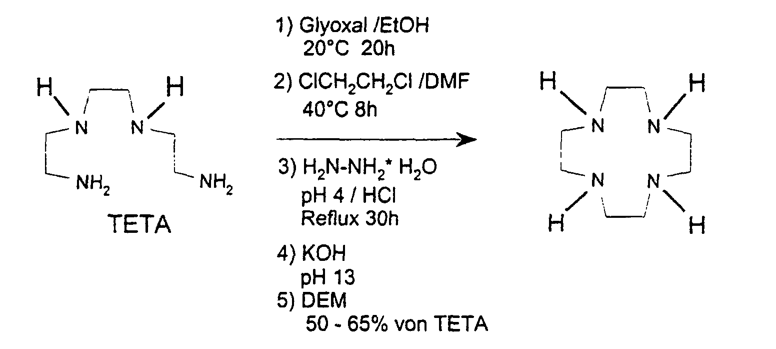

### Vorteile des Verfahrens:

Das erfindungsgemäße Verfahren zur Herstellung von Cyclen weist auf Grund seiner Auslegung als Eintopfverfahren gegenüber den bisherigen Verfahren deutliche Vorteile auf.
- Es sind keine zeit- und rohstoffintensiven Isolierungsschritte von Zwischenprodukten erforderlich.
- Die Umsetzung mit dem Amin erfolgt, ohne nennenswerte Mengen an Nebenprodukten zu generieren.
- Die Rohstoffe sind preiswert und leicht zugänglich.
- Es fallen wenig Abfälle an.
- Die Gesamtsynthesezeit ist kurz.
- Es wird ein neues kostengünstiges Reinigungsverfahren für Cyclen verwendet.
- Die Ausbeute ist höher als bei den Verfahren des Standes der Technik.

Die nachfolgenden Beispiele dienen der näheren Erläuterung des Erfindungsgegenstandes.

### Beispiel 1

### 1,4,7,10 Tetraazacyclododekan (= Cyclen):

50 g Triethylentetramin (0.342 mol) werden in 1l Ethanol gelöst und bei Raumtemperatur mit 39 ml 40%igem Glyoxal in Wasser (0.342 mol) versetzt. Nach 20 stündigem Rühren wird das Lösemittel im Vakuum abdestilliert und man erhält ein orangefarbenes Öl, das in 400 ml Dimethylformamid aufgenommen und mit 81.2 ml (101.5 g = 1.026 mol) 1,2-Dichlorethan versetzt wird. Nach 8 stündigem Rühren bei 40 °C wird im Vakuum eingeengt, der Rückstand in 400 ml Ethanol aufgenommen und mit 37%iger aq. Salzsäure auf etwa pH = 3 - 4 angesäuert. Zu dieser Reaktionslösung gibt man bei Raumtemperatur 166 ml (171 g = 3.42 mol) Hydrazinhydrat und erhitzt 30 Stunden unter Rückfluß. Die Reaktionslösung wird mit festem Kaliumhydroxyd auf pH = 13 eingestellt. Im Anschluß wird die Reaktionslösung im Vakuum eingeengt, nochmals in 100 ml Ethanol aufgenommen und das Lösemittel entfernt. Der Rückstand wird mit 25 g Aktivkohle und 100 ml Formaldehyddiethylacetal versetzt und für einige Zeit unter Rückfluß erhitzt, bevor man die heiße Lösung über eine Membran filtriert . Nach dem Erkalten der Lösung wird das Produkt durch Filtration isoliert. Man erhält 38.3 g Cyclen (0.222 mol = 65% d.Th.) als kristallinen Feststoff.

### Beispiel 2

### 1,4,7,10 Tetraazacyclododekan (= Cyclen)

50 g Triethylentetramin (0,342 mol) werden in 1 I Ethanol gelöst und bei Raumtemperatur mit 39 ml 40%igem Glyoxal in Wasser (0,342 mol) versetzt. Nach 20 stündigem Rühren wird das Lösungsmittel im Vakuum abdestilliert und man erhält ein orangefarbenes Öl, das anschließend in 400 ml Dimethylformamid aufgenommen und mit 88,5 ml (192,8 g = 1,026 mol) 1,2-Dibromethan versetzt wird. Nach 6 stündigem Rühren bei 40 °C wird im Vakuum eingeengt, der Rückstand in 400 ml Ethanol aufgenommen und mit 37 %iger aqu. Salzsäure auf pH = 3 - 4 angesäuert. Zu dieser Reaktionslösung gibt man bei Raumtemperatur 166 ml (171 g = 3,42 mol) Hydrazinhydrat und erhitzt 30 Stunden unter Rückfluß. Die Reaktionslösung wird mit festem Kaliumhydroxid auf pH = 13 eingestellt. Im Anschluß wird die Reaktionslösung im Vakuum eingeengt, nochmals in 100 ml Ethanol aufgenommen und das Lösungsmittel erneut im Vakuum entfernt. Der Rückstand wird mit 25 g Aktivkohle und 150 ml Formaldehyddiethylacetal versetzt und für einige Zeit unter Rückfluß erhitzt, bevor man die heiße Lösung über eine Membran filtriert. Nach Erkalten der Lösung wird das Produkt durch Filtration isoliert. Man erhält 39,5 g Cyclen (67 % d.Th.) als kristallinen Feststoff.

### Beispiel 3

### 1,4,7,10 Tetraazacyclododekan (= Cyclen)

50 g Triethylentetramin (0,342 mol) werden in 1 I Ethanol gelöst und bei Raumtemperatur mit 39 ml 40%igem Glyoxal in Wasser (0,342 mol) versetzt. Nach 20 stündigem Rühren wird das Lösungsmittel im Vakuum abdestilliert und man erhält ein orangefarbenes Öl, das anschließend in 400 ml Dimethylformamid aufgenommen und mit 82,6 ml (274,8 g = 1,026 mol) 1,2-Dijodethan versetzt wird. Nach 5 stündigem Rühren bei 40 °C wird im Vakuum eingeengt, der Rückstand in 400 ml Ethanol aufgenommen und mit 37 %iger aqu. Salzsäure auf pH = 3 - 4 angesäuert. Zu dieser Reaktionslösung gibt man bei Raumtemperatur 166 ml (171 g = 3,42 mol) Hydrazinhydrat und erhitzt 30 Stunden unter Rückfluß. Die Reaktionslösung wird mit festem Kaliumhydroxid auf pH = 13 eingestellt. Im Anschluß wird die Reaktionslösung im Vakuum eingeengt, nochmals in 100 ml Ethanol aufgenommen und das Lösungsmittel erneut im Vakuum entfernt. Der Rückstand wird mit 25 g Aktivkohle und 150 ml Formaldehyddiethylacetal versetzt und für einige Zeit unter Rückfluß erhitzt, bevor man die heiße Lösung über eine Membran filtriert. Nach Erkalten der Lösung wird das Produkt durch Filtration isoliert. Man erhält 37,1 g Cyclen (63 % d.Th.) als kristallinen Feststoff.

### Beispiel 4

### 1,4,7,10 Tetraazacyclododekan (= Cyclen)

50 g Triethylentetramin (0,342 mol) werden in 1 I Methanol gelöst und bei Raumtemperatur mit 39 ml 40%igem Glyoxal in Wasser (0,342 mol) versetzt. Nach 20 stündigem Rühren wird das Lösungsmittel im Vakuum abdestilliert und man erhält ein orangefarbenes Öl, das anschließend in 400 ml Dimethylformamid aufgenommen und mit 81,2 ml (101,5 g = 1,026 mol) 1,2-Dichlorethan versetzt wird. Nach 8 stündigem Rühren bei 40 °C wird im Vakuum eingeengt, der Rückstand in 400 ml Ethanol aufgenommen und mit 37 %iger aqu. Salzsäure auf pH = 3 - 4 angesäuert. Zu dieser Reaktionslösung gibt man bei Raumtemperatur 166 ml (171 g = 3,42 mol) Hydrazinhydrat und erhitzt 30 Stunden unter Rückfluß. Die Reaktionslösung wird mit festem Kaliumhydroxid auf pH = 13 eingestellt. Im Anschluß wird die Reaktionslösung im Vakuum eingeengt, nochmals in 100 ml Ethanol aufgenommen und das Lösungsmittel erneut im Vakuum entfernt. Der Rückstand wird mit 25 g Aktivkohle und 150 ml Formaldehyddiethylacetal versetzt und für einige Zeit unter Rückfluß erhitzt, bevor man die heiße Lösung über eine Membran filtriert. Nach Erkalten der Lösung wird das Produkt durch Filtration isoliert. Man erhält 37,7 g Cyclen (64 % d.Th.) als kristallinen Feststoff.

### Beispiel 5

### 1,4,7,10 Tetraazacyclododekan (= Cyclen)

50 g Triethylentetramin (0,342 mol) werden in 1 I Ethanol gelöst und bei Raumtemperatur mit 39 ml 40%igem Glyoxal in Wasser (0,342 mol) versetzt. Nach 20 stündigem Rühren wird das Lösungsmittel im Vakuum abdestilliert und man erhält ein orangefarbenes Öl, das anschließend in 400 ml Dimethylacetamid aufgenommen und mit 81,2 ml (101,5 g = 1,026 mol) 1,2-Dichlorethan versetzt wird. Nach 8 stündigem Rühren bei 40 °C wird im Vakuum eingeengt, der Rückstand in 400 ml Ethanol aufgenommen und mit 37 %iger aqu. Salzsäure auf pH = 3 - 4 angesäuert. Zu dieser Reaktionslösung gibt man bei Raumtemperatur 166 ml (171 g = 3,42 mol) Hydrazinhydrat und erhitzt 30 Stunden unter Rückfluß. Die Reaktionslösung wird mit festem Kaliumhydroxid auf pH = 13 eingestellt. Im Anschluß wird die Reaktionslösung im Vakuum eingeengt, nochmals in 100 ml Ethanol aufgenommen und das Lösungsmittel erneut im Vakuum entfernt. Der Rückstand wird mit 25 g Aktivkohle und 150 ml Formaldehyddiethylacetal versetzt und für einige Zeit unter Rückfluß erhitzt, bevor man die heiße Lösung über eine Membran filtriert. Nach Erkalten der Lösung wird das Produkt durch Filtration isoliert. Man erhält 37,7 g Cyclen (64 % d.Th.) als kristallinen Feststoff.

### Beispiel 6

### 1,4,7,10 Tetraazacyclododekan (= Cyclen)

50 g Triethylentetramin (0,342 mol) werden in 1 I Ethanol gelöst und bei Raumtemperatur mit 39 ml 40 %igem Glyoxal in Wasser (0,342 mol) versetzt. Nach 20 stündigem Rühren wird das Lösungsmittel im Vakuum abdestilliert und man erhält ein orangefarbenes Öl, das anschließend in 400 ml Tetramethylharnstoff aufgenommen und mit 81,2 ml (101,5 g = 1,026 mol) 1,2-Dichlorethan versetzt wird. Nach 8 stündigem Rühren bei 40 °C wird im Vakuum eingeengt, der Rückstand in 400 ml Ethanol aufgenommen und mit 37 %iger aqu. Salzsäure auf pH = 3 - 4 angesäuert. Zu dieser Reaktionslösung gibt man bei Raumtemperatur 166 ml (171 g = 3,42 mol) Hydrazinhydrat und erhitzt 30 Stunden unter Rückfluß. Die Reaktionslösung wird mit festem Kaliumhydroxid auf pH = 13 eingestellt. Im Anschluß wird die Reaktionslösung im Vakuum eingeengt, nochmals in 100 ml Ethanol aufgenommen und das Lösungsmittel erneut im Vakuum entfernt. Der Rückstand wird mit 25 g Aktivkohle und 150 ml Formaldehyddiethylacetal versetzt und für einige Zeit unter Rückfluß erhitzt, bevor man die heiße Lösung über eine Membran filtriert. Nach Erkalten der Lösung wird das Produkt durch Filtration isoliert. Man erhält 37,1 g Cyclen (63 % d.Th.) als kristallinen Feststoff.

### Beispiel 7

### 1,4,7,10 Tetraazacyclododekan (= Cyclen)

50 g Triethylentetramin (0,342 mol) werden in 1 I Ethanol gelöst und bei Raumtemperatur mit 39 ml 40%igem Glyoxal in Wasser (0,342 mol) versetzt. Nach 20 stündigem Rühren wird das Lösungsmittel im Vakuum abdestilliert und man erhält ein orangefarbenes Öl, das anschließend in 400 ml Tetramethylharnstoff aufgenommen und mit 88,5 ml (192,8 g = 1,026 mol) 1,2-Dibromethan versetzt wird. Nach 6 stündigem Rühren bei 40 °C wird im Vakuum eingeengt, der Rückstand in 400 ml Ethanol aufgenommen und mit 37 %iger aqu. Salzsäure auf pH = 3 - 4 angesäuert. Zu dieser Reaktionslösung gibt man bei Raumtemperatur 166 ml (171 g = 3,42 mol) Hydrazinhydrat und erhitzt 30 Stunden unter Rückfluß. Die Reaktionslösung wird mit festem Kaliumhydroxid auf pH = 13 eingestellt. Im Anschluß wird die Reaktionslösung im Vakuum eingeengt, nochmals in 100 ml Ethanol aufgenommen und das Lösungsmittel erneut im Vakuum entfernt. Der Rückstand wird mit 25 g Aktivkohle und 150 ml Formaldehyddiethylacetal versetzt und für einige Zeit unter Rückfluß erhitzt, bevor man die heiße Lösung über eine Membran filtriert. Nach Erkalten der Lösung wird das Produkt durch Filtration isoliert. Man erhält 37,6 g Cyclen (64 % d.M.) als kristallinen Feststoff.

### Beispiel 8

### 1,4,7, 10 Tetraazacyclododekan (= Cyclen)

50 g Triethylentetramin (0,342 mol) werden in 1 I Methanol gelöst und bei Raumtemperatur mit 39 ml 40 %igem Glyoxal in Wasser (0,342 mol) versetzt. Nach 20 stündigem Rühren wird das Lösungsmittel im Vakuum abdestilliert und man erhält ein orangefarbenes Öl, das anschließend in 400 ml Dimethylformamid aufgenommen und mit 88,5 ml (192,8 g = 1,026 mol) 1,2-Dibromethan versetzt wird. Nach 6 stündigem Rühren bei 40 °C wird im Vakuum eingeengt, der Rückstand in 400 ml Ethanol aufgenommen und mit 37 %iger aqu. Salzsäure auf pH = 3 - 4 angesäuert. Zu dieser Reaktionslösung gibt man bei Raumtemperatur 166 ml (171 g = 3,42 mol) Hydrazinhydrat und erhitzt 30 Stunden unter Rückfluß. Die Reaktionslösung wird mit festem Kaliumhydroxid auf pH = 13 eingestellt. Im Anschluß wird die Reaktionslösung im Vakuum eingeengt, nochmals in 100 ml Ethanol aufgenommen und das Lösungsmittel erneut im Vakuum entfernt. Der Rückstand wird mit 25 g Aktivkohle und 150 ml Formaldehyddiethylacetal versetzt und für einige Zeit unter Rückfluß erhitzt, bevor man die heiße Lösung über eine Membran filtriert. Nach Erkalten der Lösung wird das Produkt durch Filtration isoliert. Man erhält 35,9 g Cyclen (61 % d.Th.) als kristallinen Feststoff.

### Beispiel 9

### 1,4,7,10 Tetraazacyclododekan (= Cyclen)

50 g Triethylentetramin (0,342 mol) werden in 1 I Ethanol gelöst und bei Raumtemperatur mit 39 ml 40%igem Glyoxal in Wasser (0,342 mol) versetzt. Nach 20 stündigem Rühren wird das Lösungsmittel im Vakuum abdestilliert und man erhält ein orangefarbenes Öl, das anschließend in 400 ml Dimethylformamid aufgenommen und mit 81,2 ml (101,5 g = 1,026 mol) 1,2-Dichlorethan versetzt wird. Nach 8 stündigem Rühren bei 40 °C wird im Vakuum eingeengt, der Rückstand in 400 ml Ethanol aufgenommen und mit 37 %iger aqu. Salzsäure auf pH = 3 - 4 angesäuert. Zu dieser Reaktionslösung gibt man bei Raumtemperatur 166 ml (171 g = 3,42 mol) Hydrazinhydrat und erhitzt 30 Stunden unter Rückfluß. Die Reaktionslösung wird mit festem Kaliumhydroxid auf pH = 13 eingestellt. Im Anschluß wird die Reaktionslösung im Vakuum eingeengt, nochmals in 100 ml Ethanol aufgenommen und das Lösungsmittel erneut im Vakuum entfernt. Der Rückstand wird mit 25 g Aktivkohle und 200 ml Toluol versetzt und für einige Zeit unter Rückfluß erhitzt, bevor man die heiße Lösung über eine Membran filtriert. Nach Erkalten der Lösung wird das Produkt durch Filtration isoliert. Man erhält 35,8 g Cyclen (61 % d.Th.) als kristallinen Feststoff.

### Beispiel 10

### 1,4,7,10 Tetraazacyclododekan (= Cyclen)

50 g Triethylentetramin (0,342 mol) werden in 1 I 2-Propanol gelöst und bei Raumtemperatur mit 39 ml 40%igem Glyoxal in Wasser (0,342 mol) versetzt. Nach 20 stündigem Rühren wird das Lösungsmittel im Vakuum abdestilliert und man erhält ein orangefarbenes Öl, das anschließend in 400 ml Dimethylformamid aufgenommen und mit 81,2 ml (101,5 g = 1,026 mol) 1,2-Dichlorethan versetzt wird. Nach 8 stündigem Rühren bei 40 °C wird im Vakuum eingeengt, der Rückstand in 400 ml Ethanol aufgenommen und mit 37 %iger aqu. Salzsäure auf pH = 3 - 4 angesäuert. Zu dieser Reaktionslösung gibt man bei Raumtemperatur 166 ml (171 g = 3,42 mol) Hydrazinhydrat und erhitzt 30 Stunden unter Rückfluß. Die Reaktionslösung wird mit festem Kaliumhydroxid auf pH = 13 eingestellt. Im Anschluß wird die Reaktionslösung im Vakuum eingeengt, nochmals in 100 ml Ethanol aufgenommen und das Lösungsmittel erneut im Vakuum entfernt. Der Rückstand wird mit 25 g Aktivkohle und 150 ml Formaldehyddiethylacetal versetzt und für einige Zeit unter Rückfluß erhitzt, bevor man die heiße Lösung über eine Membran filtriert. Nach Erkalten der Lösung wird das Produkt durch Filtration isoliert. Man erhält 37,2g Cyclen (63 % d.Th.) als kristallinen Feststoff.

## Patentansprüche

1. Verfahren zur Herstellung von Cyclen 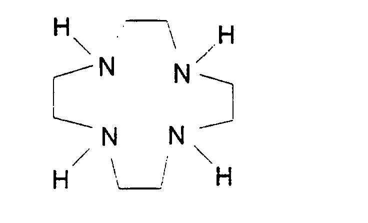 **dadurch gekennzeichnet, daß** in einem Eintopfverfahren Triethylentetramin mit 40 %igem Glyoxal bei 20 ° C bis 80 ° C in einem polaren protischen Lösungsmittel innerhalb von 4 bis 40 Stunden umgesetzt wird, der so gebildete intermediäre Tricyclus nach Entfernen des Lösungsmittels mit einem 1,2-difunktionalisierten Alkylierungsmittel X(CH₂)₂X, worin X für eine nucleofuge Gruppe steht, in einem polaren aprotischen Lösungsmittel, gegebenenfalls in Gegenwart einer Hilfsbase, bei 20 bis 120 ° C innerhalb von 2 bis 24 Stunden an den beiden sekundären Amin-Stickstoffen alkyliert wird, das so erhaltene Kondensationsprodukt nach Entfernen des Lösungsmittels mit Hydrazinhydrat in einem polaren protischen Lösungsmittel bei einem pH von 3 bis 6 innerhalb von 12 bis 48 Stunden bei Rückflußtemperatur behandelt wird, anschließend das Cyclen durch Zugabe einer Base aus dem Cyclensalz freigesetzt wird und nach Eindampfen der Reaktionslösung zur Trockne isoliert wird.

2. Verfahren nach Anspruch 1, **dadurch gekennzeichnet, daß** als polare protische Lösungsmittel Methanol, Ethanol, Isopropanol, Butanol, Glykol, Wasser oder deren Mischungen verwendet werden.

3. Verfahren nach Anspruch 1, **dadurch gekennzeichnet, daß** als polare aprotische Lösungsmittel N,N-Dimethylformamid (DMF), N,N-Dimethylacetamid (DMAC), N-Methylpyrrolidon (NMP), Tetramethylharnstoff, Formamid oder Dimethylpropylenharnstoff (DMPU) verwendet werden.

4. Verfahren nach Anspruch 1, **dadurch gekennzeichnet, daß** als Alkylierungsmittel X(CH₂)₂X 1,2-Dibromethan, 1,2-Dichlorethan, 1,2-Ditosylethan, 1,2-Dimesylethan oder 1,2-Diiodethan verwendet wird.

5. Verfahren nach Anspruch 1, **dadurch gekennzeichnet, daß** als bei der Kondensationsreaktion mit dem 1,2-difunktionalisierten Alkylierungsmittel X(CH₂)₂X gegebenenfalls verwendeten Hilfsbase Natriumcarbonat, Kaliumcarbonat, Calciumcarbonat, Natriumhydrogencarbonat, Kaliumhydrogencarbonat, Magnesiumcarbonat, Magnesiumhydrogencarbonat, Lithiumhydroxid oder Lithiumcarbonat dient.

6. Verfahren nach Anspruch 1, **dadurch gekennzeichnet, daß** als Base zur Freisetzung des Cyclens Natriumhydroxid, Kaliumhydroxid, Calciumhydroxid oder ein basischer lonenaustauscher verwendet wird.

7. Verfahren nach Anspruch 1, **dadurch gekennzeichnet, daß** die Umsetzung des Triethylentetraamins mit Glyoxal bei 20 bis 40° C innerhalb von 15 bis 20 Stunden durchgeführt wird.

8. Verfahren nach Anspruch 1, **dadurch gekennzeichnet, daß** die Kondensationsreaktion mit dem 1,2-difunktionalisierten Alkylierungsmittel X(CH₂)₂X bei 30 bis 70 °C und innerhalb von 6 bis 10 Stunden durchgeführt wird.

9. Verfahren nach Anspruch 1, **dadurch gekennzeichnet, daß** die Umsetzung des Kondensationsprodukts mit Hydrazinhydrat innerhalb von 25 bis 35 Stunden durchgeführt wird.

10. Verfahren nach Anspruch 1, **dadurch gekennzeichnet, daß** das Reaktionsprodukt durch Behandeln des nach Eindampfen der Reaktionslösung erhaltenen Rückstands mit Toluol, Trifluormethylbenzol oder Diethoxymethan isoliert wird.

## Claims

1. Process for the production of cyclene 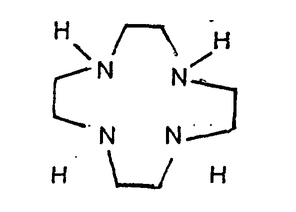 **characterized in that** in a single-pot process, triethylenetetramine is reacted with 40% glyoxal at 20°C. to 80°C in a polar, protic solvent within 4 to 40 hours; after the solvent has been removed, the intermediate tricyclic compound that is thus formed is alkylated to the two secondary amine-nitrogens with a 1,2-difunctionalized alkylating agent X(CH₂)₂x, in which X stands for a nucleofuge group, in a polar aprotic solvent, optionally in the presence of an auxiliary base, at 20 to 120°C within 2 to 24 hours; after the solvent has been removed, the thus obtained condensation product is treated with hydrazine hydrate in a polar protic solvent at a pH of 3 to 6 within 12 to 48 hours at reflux temperature; then the cyclene is released from the cyclene salt by adding a base, and after the reaction solution is evaporated to the dry state, it is isolated.

2. Process according to Claim 1, **characterized in that** methanol, ethanol, isopropanol, butanol, glycol, water or mixtures thereof are used as polar protic solvents.

3. Process according to Claim 1, **characterized in that** N,N-dimethylformamide (DMF), N,N-dimethylacetamide (DMAC), N-methylpyrrolidone (NMP), tetramethylurea, formamide or dimethylpropyleneurea (DMPU) is used as a polar aprotic solvent.

4. Process according to Claim 1, **characterized in that** 1,2-dibromoethane, 1,2-dichloroethane, 1,2-ditosylethane, 1,2-dimesylethane or 1,2-diiodoethane is used as an alkylating agent X(CH₂)₂X.

5. Process according to Claim 1, **characterized in that** sodium carbonate, potassium carbonate, calcium carbonate, sodium bicarbonate, potassium hydrogen carbonate, magnesium carbonate, magnesium hydrogen carbonate, lithium hydroxide or lithium carbonate is used as an auxiliary base that is optionally used in the condensation reaction with the 1,2-difunctionalized alkylating agent X(CH₂)₂X.

6. Process according to Claim 1, **characterized in that** sodium hydroxide, potassium hydroxide, calcium hydroxide or a basic ion exchanger is used as a base to release cyclene.

7. Process according to Claim 1, **characterized in that** the reaction of triethylenetetramine with glyoxal is performed at 20 to 40°C within 15 to 20 hours.

8. Process according to Claim 1, **characterized in that** the condensation reaction is performed with the 1,2-difunctionalized alkylating agent X(CH₂)₂X at 30 to 70°C and within 6 to 10 hours.

9. Process according to Claim 1, **characterized in that** the reaction of the condensation product with hydrazine hydrate is performed within 25 to 35 hours.

10. Process according to Claim 1, **characterized in that** the reaction product is isolated by treating with toluene, trifluoromethylbenzene or diethoxymethane the residue that is obtained after the reaction solution has been concentrated by evaporation.

## Revendications

1. Procédé de préparation de cyclène 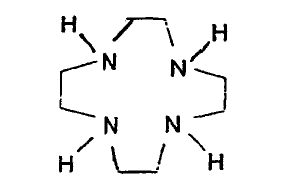 **caractérisé en ce que**, dans un procédé en un seul pot, de la triéthylènetétramine est mise à réagir avec du glyoxal à 40 % entre 20°C et 80°C dans un solvant protique polaire en l'espace de 4 à 40 heures, le tricycle intermédiaire ainsi formé est alkylé sur les deux azotes aminiques secondaires, après élimination du solvant, avec un agent d'alkylation 1,2-difonctionnalisé X(CH₂)₂X, dans lequel X représente un groupe nucléofuge, dans un solvant aprotique polaire, éventuellement en présence d'une base auxiliaire, entre 20 et 120°C et en l'espace de 2 à 24 heures, le produit de condensation ainsi obtenu est traité avec de l'hydrate d'hydrazine, après élimination du solvant, dans un solvant protique polaire à un pH de 3 à 6 à la température de reflux, en l'espace de 12 à 48 heures, puis le cyclène est libéré du sel de cyclène par addition d'une base et isolé après évaporation à sec de la solution réactionnelle.

2. Procédé selon la revendication 1, **caractérisé en ce que** l'on utilise, en tant que solvants protiques polaires, le méthanol, l'éthanol, l'isopropanol, le butanol, le glycol, l'eau ou leurs mélanges.

3. Procédé selon la revendication 1, **caractérisé en ce que** l'on utilise, en tant que solvants aprotiques polaires, le N,N-diméthylformamide (DMF), le N,N-diméthylacétamide (DMAC), la N-méthylpyrrolidone (NMP), la tétraméthylurée, le formamide ou la diméthylpropylène-urée (DMPU).

4. Procédé selon la revendication 1, **caractérisé en ce que** l'on utilise, en tant qu'agents d'alkylation X(CH₂)₂X, le 1,2-dibromoéthane, le 1,2-dichloroéthane, le 1,2-ditosyléthane, le 1,2-dimésyléthane ou le 1,2-diiodoéthane.

5. Procédé selon la revendication 1, **caractérisé en ce que** l'on utilise, en tant que base auxiliaire éventuellement utilisée lors de la réaction de condensation avec l'agent d'alkylation 1,2-difonctionnalisé X(CH₂)₂X, le carbonate de sodium, le carbonate de potassium, le carbonate de calcium, l'hydrogénocarbonate de sodium, l'hydrogénocarbonat de potassium, le carbonate de magnésium, l'hydrogénocarbonat de magnésium, l'hydroxyde de lithium ou le carbonate de lithium.

6. Procédé selon la revendication 1, **caractérisé en ce que** l'on utilise, en tant que base pour la libération du cyclène, l'hydroxyde de sodium, l'hydroxyde de potassium, l'hydroxyde de calcium ou un échangeur d'ions basique.

7. Procédé selon la revendication 1, **caractérisé en ce que** la réaction de la triéthylènetétramine avec le glyoxal est réalisée entre 20 et 40°C en l'espace de 15 à 20 heures.

8. Procédé selon la revendication 1, **caractérisé en ce que** la réaction de condensation avec l'agent d'alkylation 1,2-difonctionnalisé X(CH₂)₂X est réalisée entre 30 et 70°C et en l'espace de 6 à 10 heures.

9. Procédé selon la revendication 1, **caractérisé en ce que** la réaction du produit de condensation avec l'hydrate d'hydrazine est réalisée en l'espace de 25 à 35 heures.

10. Procédé selon la revendication 1, **caractérisé en ce que** le produit de réaction est isolé par traitement du résidu obtenu après évaporation de la solution réactionnelle, avec du toluène, du trifluorométhylbenzène ou du diéthoxyméthane.
